(19) Europäisches Patentamt

European Patent Office

Office européen des brevets

(11) EP 0 739 619 B1

(12) **FASCICULE DE BREVET EUROPEEN**

(45) Date de publication et mention de la délivrance du brevet:
**14.01.1998 Bulletin 1998/03**

(51) Int. Cl.⁶: **A61K 7/00**, A61K 7/50, A61K 7/48

(21) Numéro de dépôt: **96400706.6**

(22) Date de dépôt: **01.04.1996**

(54) **Emulsion huile-dans-eau moussante à base de tensio-actifs non-ioniques, d'une phase grasse et d'un polymère cationique ou anionique réticulé et utilisation en application topique**

Schäumende Emulsion auf der Basis von nicht-ionischen Tensiden, einer Fettphase und einem vernetzten anionischen oder kationischen Polymer zur topischen Anwendung

Foaming o/w emulsion based on non-ionic surfactants, a fatty phase and a cross-linked cationic or anionic polymer for topical use

(84) Etats contractants désignés:
**DE ES FR GB IT**

(30) Priorité: **25.04.1995 FR 9504924**

(43) Date de publication de la demande:
**30.10.1996 Bulletin 1996/44**

(73) Titulaire: **L'OREAL**
**75008 Paris (FR)**

(72) Inventeurs:
  • **Sebillotte-Arnaud, Laurence**
  **94000 Creteil (FR)**
  • **Ambrosini, Emmanuelle**
  **75013 Paris (FR)**
  • **Arnaud, Pascal**
  **94000 Creteil (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
  **EP-A- 0 617 954**          **EP-A- 0 642 781**
  **WO-A-92/07543**          **WO-A-95/03781**
  **WO-A-95/11000**          **WO-A-95/31978**
  **FR-A- 2 689 010**          **FR-A- 2 720 934**

  • **JOURNAL OF PAINT TECHNOLOGY, vol. 39, no. 504, 1967, pages 104-117, XP002010040 C. M. HANSEN: "The Three Dimensional Solubility Parameter-Key to Paint Component Affinities"**

## Description

La présente invention a pour objet des émulsions huile-dans-eau moussantes sous forme de crème à base de tensio-actifs non-ioniques, d'une phase grasse spécifique et d'un polymère réticulé constitué d'un monomère cationique ou anionique à insaturation éthylénique et leurs utilisations en application topique notamment en cosmétique et en dermatologie.

Les crèmes moussantes de nettoyage de la peau, douces au toucher, se présentent sous forme d'émulsion huile-dans-eau contenant des tensio-actifs détergents et moussants. La phase huileuse permet d'adoucir la peau après rinçage. Cependant, la phase huileuse a tendance à inhiber les propriétés moussantes de ces formulations ; on dit qu'elle "casse" la mousse. On l'introduit par conséquent dans les crèmes moussantes à de faibles concentrations, en général inférieures à 5% en poids.

Les systèmes tensio-actifs utilisés dans les crèmes moussantes sont généralement constitués principalement de tensio-actifs anioniques. Malgré leur pouvoir moussant élevé et leur bonne détergence, les tensio-actifs anioniques présentent un certain potentiel irritant vis-à-vis de la peau et des yeux et sont incompatibles avec un grand nombre d'agents gélifiants.

Pour réduire ce potentiel irritant, on associe aux tensio-actifs anioniques des tensio-actifs amphotères et/ou des tensio-actifs non-ioniques. Ainsi, la demande de brevet WO 93/19149 décrit des compositions moussantes de nettoyage contenant un système tensio-actif constitué de tensio-actifs anioniques associés à des tensio-actifs amphotères en présence d'une huile non-soluble dans l'eau et/ou d'une cire.

Le principal objectif de l'invention est de pouvoir réaliser des crèmes moussantes à base d'un système tensio-actif moussant exclusivement non-ionique, pouvant contenir d'importantes quantités d'huile sans nuire pour autant au pouvoir moussant.

L'art antérieur enseigne que lorsque l'on prépare des compositions à base de tensioactifs non-ioniques et d'huile, le pouvoir moussant de ces compositions chute lorsque l'on augmente la proportion d'huile par rapport au système tensio-actif.

Un tel enseignement est illustré en particulier par le document EP-617954, qui décrit des compositions cosmétiques moussantes comprenant au moins un agent tensio-actif non-ionique du type alkylpolyglucoside et/ou polyglycérolé et au moins un copolymère réticulé d'anhydride maléique/alkyl-vinyl éther et éventuellement une huile dans de très faibles proportions. Ce document ne décrit pas de crèmes moussantes.

Le document EP-642781 illustre un autre aspect de ce même principe en décrivant des crèmes non-moussantes, acides, sous la forme d'émulsions huile-dans-eau, stables, comprenant un système tensio-actif non-ionique, d'importantes quantités d'huile et à titre d'agent stabilisant, des copolymères acrylamide/acide acrylamido-2 propane sulfonique réticulés. Les compositions décrites dans ce document ne moussent pas.

On connaît par ailleurs, par le document US-5,011,681, des compositions cosmétiques pour le nettoyage de la peau, sous la forme d'émulsions huile-dans-eau comprenant un tensio-actif de HLB supérieur ou égal à 10, une phase huileuse comprenant une polyalphaoléfine et un copolymère carboxylique comportant 95,9 à 98,8% en poids de monomère monooléfiniquement insaturé, choisi parmi les acides acrylique, méthacrylique et éthacrylique et 1 à 3,5% d'un ester d'acide acrylique méthacrylique ou éthacrylique avec un alcool en $C_{10}$-$C_{30}$. Ces compositions présentent l'inconvénient d'avoir un toucher gluant, un aspect filant et un comportement élastique. En outre, leur pouvoir moussant est insuffisant. Les compositions selon la présente invention remédient à ces inconvénients de l'art antérieur.

La demanderesse a découvert de manière surprenante que l'objectif qu'elle s'était fixé pouvait être atteint par de nouvelles compositions aqueuses moussantes sous forme de crème contenant un système tensio-actif exclusivement non-ionique, une phase grasse présentant des caractéristiques particulières qui seront définies ci-après, cette phase grasse pouvant être présente en quantité importante par rapport au système tensio-actif, et un polymère ou copolymère réticulé d'un monomère, cationique ou anionique, à insaturation éthylénique.

Les crèmes moussantes conformes à l'invention sont stables au stockage, présentent d'excellentes propriétés moussantes, une meilleure tolérance et innocuité vis-à-vis de la peau et des yeux par rapport aux compositions moussantes connues, et de bonnes propriétés cosmétiques à la prise et au toucher.

En outre, les compositions de l'invention peuvent contenir des quantités d'huiles importantes pouvant atteindre jusqu'à 6 à 10 fois la quantité de tensio-actifs non-ioniques présents dans la formulation, sans affecter de manière significative, le pouvoir moussant et la stabilité des compositions.

Il est ainsi possible d'introduire dans de telles compositions des quantités importantes d'huile de soin et d'obtenir des produits moussants "deux en un".

Les compositions selon l'invention sont caractérisées par le fait qu'elles comprennent, dans un milieu aqueux cosmétiquement acceptable,

a) 5 à 20%, en poids par rapport au poids total de la composition, d'un système tensio-actif exclusivement non-ionique comprenant au moins un tensio-actif non-ionique moussant ;

b) 10 à 50%, en poids par rapport au poids total de la composition, d'une phase huileuse comportant au moins une huile insoluble dans l'eau dont les paramètres de solubilité dD, dP et dH selon l'espace de solubilité de HANSEN satisfont aux conditions suivantes :

$$15,5 \ (J/cm^3)^{\frac{1}{2}} \leq dD \leq 18,0 \ (J/cm^3)^{\frac{1}{2}}$$

$$et \ 0 \ (J/cm^3)^{\frac{1}{2}} \leq dA \leq 6,5 \ (J/cm^3)^{\frac{1}{2}}$$

$$avec \ dA = (dP^2 + dH^2)^{\frac{1}{2}}$$

c) et comme agent gélifiant, au moins un homopolymère ou copolymère réticulé, formé à partir d'au moins un monomère, cationique ou anionique, à insaturation éthylénique et d'un agent de réticulation à polyinsaturation éthylénique, à l'exception des copolymères comportant une fraction majoritaire d'au moins un acide oléfiniquement insaturé en $C_3$-$C_6$ et une fraction minoritaire d'au moins un ester d'un acide oléfiniquement insaturé en $C_3$-$C_6$ avec un alcool en $C_{10}$-$C_{30}$.

Préférentiellement, les compositions selon l'invention sont caractérisées par le fait qu'elles comprennent, dans un milieu aqueux cosmétiquement acceptable,

a) 5 à 10%, en poids par rapport au poids total de la composition, d'un système tensio-actif exclusivement non-ionique comprenant au moins un tensio-actif non-ionique moussant ;

b) 10 à 30%, en poids par rapport au poids total de la composition, d'une phase huileuse comportant au moins une huile insoluble dans l'eau dont les paramètres de solubilité sont tels que défini ci-dessus.

Les tensio-actifs non-ioniques moussants utilisés selon la présente invention sont choisis parmi les tensio-actifs non-ioniques possédant un pouvoir moussant caractérisé par une hauteur de mousse supérieure à 100 mm ; de préférence, supérieure à 120 mm mesurée selon la méthode ROSS-MILES pour une solution à 0,1 % en poids de tensio-actif dans de l'eau distillée à 25° C.

Les tensio-actifs non-ioniques moussants utilisés dans les compositions selon l'invention sont plus particulièrement choisis parmi :

(i) les composés de formule :

$$R—CHOH—CH_2—O[CH_2CHOH—CH_2O]_nH \tag{I}$$

dans laquelle R désigne un radical ou un mélange de radicaux aliphatiques, cycloaliphatiques ou arylaliphatiques, ayant de 7 à 21 atomes de carbone, les chaînes aliphatiques pouvant être saturées ou non-saturées, linéaires ou ramifiées et pouvant comporter des atomes de souffre ou d'oxygène et en particulier des groupements éther, thioéther ou hydroxyméthylène ; n est un nombre quelconque, entier ou décimal supérieur à 1 inférieur ou égal à 10 et désigne le degré de polymérisation moyen ;

(ii) les composés de formule :

$$R^1 O (G)_p \tag{II}$$

dans laquelle $R^1$ est une chaîne hydrocarbonée monovalent contenant de 1 à 30 atomes de carbone, G est un dérivé de saccharide contenant de 5 à 6 atomes de carbone et p est une valeur moyenne statistique allant de 1 à 6 ;

(iii) leurs mélanges.

Les composés de formule (I) sont décrits dans le brevet FR 2 091 516. Ils sont obtenus par polycondensation d'un α-diol à chaîne grasse sur le glycidol.

Les composés préférentiels de formule (I) sont ceux pour lesquels R désigne un radical alkyle ou un mélange de radicaux alkyle comportant de 9 à 12 atomes de carbone et n représente une valeur statistique moyenne allant de 3 à 4.

On utilise en particulier le dodécanediol polyglycérolé à 3,5 moles de glycérol vendu sous la dénomination CHIMEXANE NF par la Société CHIMEX.

Les composés de formule (II) sont décrits notamment dans la demande WO 94/27562. Les composés préférentiels sont ceux pour lesquels le radical $R^1$ est un radical alkyle en $C_8$-$C_{16}$ et plus particulièrement ceux dont le degré de polymérisation est 1,4 ou 1,6.

On peut citer par exemple :

- les alkylpolyglycosides commercialisés par HENKEL sous les noms APG 225, APG 425, APG 625 ;

- les alkylpolyglucosides commercialisés par HENKEL sous le nom GLUCOPON proche de l'APG 625 avec un degré de polymérisation différent ;

- les alkylpolyglycosides commercialisés par HENKEL sous le nom PLANTAREN tels que le PLANTAREN 2000 qui est un $C_8$-$C_{16}$-alkyl-polyglucoside avec un degré de polymérisation de 1,4 ; le PLANTAREN 1200 et qui est un $C_{12}$-$C_{18}$-alkyl-polyglucoside de degré de polymérisation 1,4 ; le PLANTAREN 1300, qui est un $C_{12}$-$C_{18}$-alkylpolyglycoside de degré de polymérisation 1,6.

Les compositions selon l'invention peuvent contenir en plus des tensio-actifs non-ioniques siliconés moussants tels que les polydiméthyl-siloxanes à groupement glucoside comme le SLM SPG 120 de la société WACKER ou les dérivés de polydiméthylsiloxane à groupement alkylphosphobétaines tels que le PECOSIL SPB-1240 de la société U.C.I.B.

La définition des solvants dans l'espace de solubilité tridimensionnel selon HANSEN est décrite dans l'article de C. M. HANSEN : "The three dimensionnal solubility parameters" J. Paint Technol. 39, 105 (1967).

- dD caractérise les forces de dispersion de LONDON issues de la formation de dipôles induits lors des chocs moléculaires.

- dP caractérise les forces d'interactions de DEBYE entre dipôles permanents.

- dH caractérise les forces d'interactions spécifiques (type liaisons hydrogène, acide/base, donneur/accepteur, etc...).

- dA est déterminé par l'équation :

$$dA = (dP^2 + dH^2)^{1/2}$$

Les paramètres dD, dP, dH et dA sont exprimés en $(J/cm^3)^{1/2}$.

Selon la présente invention, la phase huileuse comprend au moins une huile telle que $15,5 \leq dD \leq 18,0$ et $0 \leq dA \leq 6,5$. De façon préférentielle, les paramètres dD et dA satisfont à la condition : $16,0 \leq dD \leq 18,0$ et $0 \leq dA \leq 5,5$.

Parmi les huiles satisfaisant aux inégalités : $15,5 \leq dD \leq 18,0$ et $O \leq dA \leq 6,5$, on peut citer, par exemple :

- les huiles minérales comme l'huile de paraffine, l'huile de vaseline.

- les huiles d'origine animale comme le perhydrosqualène.

- les huiles d'origine végétale telles l'huile de jojoba, l'huile de sésame, l'huile de colza, le beurre de karité et sa fraction liquide.

- les huiles de synthèse telles que les esters gras comme l'huile de Purcellin, le palmitate d'éthyl-2 hexyle, le stéarate d'octyl-2 dodécyle, l'érucate d'octyl-2 dodécyle, l'isostéarate d'isostéaryle et le benzoate d'octyl-2 dodecyle et l'isoparaffine (6-8 moles d'isobutylène) hydrogénée.

La phase huileuse des compositions selon l'invention peut également contenir d'autres types d'huiles qui ne satisfont pas aux conditions des paramètres de solubilité selon HANSEN déterminées ci-dessus telles que par exemple : les triglycérides des acides caprylique/caprique, l'octyl-dodécanol, l'isohexadécane.

Ces autres huiles sont, de préférence, présentes dans la phase huileuse à des concentrations inférieures ou égales à 15 % en poids par rapport au poids total de la phase huileuse. La phase huileuse comprend donc de 85 à 100 % d'huile répondant aux conditions relatives aux paramètres dD et dA définies ci-dessus.

Les homopolymères ou copolymères réticulés utilisés dans les compositions selon l'invention comprennent au moins un monomère cationique ou anionique à insaturation éthylénique et un agent de réticulation à polyinsaturation éthylénique.

Le monomère anionique est choisi en particulier parmi l'acide (meth)acrylique, le (meth)acrylate d'ammonium et l'acide 2-acrylamido-2-méthylpropane sulfonique ainsi que ses sels.

Le monomère cationique est choisi en particulier parmi les dialkylaminoalkyl-(meth)acrylates, de préférence, le diméthylamino-éthylmethacrylate ; les dialkylaminoalkyl-(meth)acrylamides ainsi que leurs sels quaternaires ou acides ; les radicaux alkyle étant, de préférence, en $C_1$-$C_4$.

Les copolymères réticulés selon l'invention comprenant de préférence un co-monomère non-ionique, choisi parmi le méthacrylamide, l'acrylamide et les esters d'acide (meth)acrylique en $C_{10}$-$C_{30}$.

Les agents de réticulation à polyinsaturation éthylénique sont choisis, de préférence, parmi le divinylbenzène ; le tétraallyloxyéthane ; l'éther diallylique ; les éthers polyallyliques polyglycéryliques ; les éthers allyliques d'alcools de la série du sucre comme l'érythrytol, le pentaérythrytol, l'arabitol, le sorbitol ou le glucose ; le méthylène-bis-acrylamide, l'éthylèneglycol di-(méth)acrylate, le di-(méth)acrylamide, le cyanométhylacrylate, le vinyloxyéthyl-(meth)acrylate ou leurs sels métalliques.

Les homopolymères ou copolymères réticulés conformes à l'invention sont choisis préférentiellement parmi :

(a) les homopolymères d'acide acrylique réticulés par un éther allylique d'alcool de la série du sucre tels que les produits vendus sous les noms CARBOPOLS 980, 981, 954, 2984 et 5984 par la société GOODRICH ou les produits vendus sous les noms SYNTHALEN M et SYNTHALEN K par la société 3 VSA ;

(b) les copolymères d'acide 2-acrylamido-2-méthyl-propane sulfonique partiellement ou totalement neutralisé (par une base telle que la soude, de la potasse ou une amine) et d'acrylamide tels que le produit décrit dans l'exemple 1 du document EP-A-503 853 ;

(c) les homopolymères d'acrylate d'ammonium tels que le produit vendu sous le nom MICROSAP PAS 5193 par la société HOECHST ou les copolymères d'acrylate d'ammonium et d'acrylamide tels que le produit vendu sous le nom BOZEPOL C

(d) les homopolymères de diméthylaminoéthyl-méthacrylate quaternisé par le chlorure de méthyle tels que les produits vendus sous les noms SALCARE 95 et SALCARE 96 par la société ALLIED COLLOIDS ou les copolymères de diméthylamino-éthylméthacrylate quaternisé par le chlorure de méthyle et d'acrylamide tel que le produit SALCARE SC92 vendu par ALLIED COLLOIDS ou le produit PAS 5194 vendu par HOECHST (ils sont décrits et préparés dans le document EP-A-395.282).

Ils sont présents dans les compositions de l'invention à des concentrations en matière active allant préférentiellement, de 0,2 à 10 % en poids, en particulier de 0,8 à 8 % en poids et plus particulièrement de 0,2 à 5 % en poids par rapport au poids total de la composition.

Les compositions selon l'invention peuvent contenir en plus des tensio-actifs non-ioniques émulsionnants tels que des alkylphénols oxyéthylénés comme le produit vendu sous le nom NIKKOL HCO (triglycérides ricinoléiques hydrogénés oxyéthylénés (60 OE)) par la société NIKKOL ou le produit vendu sous le nom SOLUBILISANT LRI (alcool butylique oxyéthyléné (26OE)) avec un mélange d'huile de ricin hydrogénée oxyéthylénée (40 OE) et d'eau (54/36) par la société WACKER. Ils sont présents, de préférence, à des concentrations allant de 0,1 à 2 % en poids et plus particulièrement de 0,5 à 1,5 % en poids.

Les compositions conformes à l'invention peuvent également contenir en plus des agents épaississants ayant un seuil d'écoulement faible à savoir pouvant s'écouler sous leur propre poids et ayant un faible pouvoir de gélification. On peut citer par exemple les gélifiants naturels tels que les galactomannanes comme le guar et le caroube ; les carraghénanes comme le D-galactopyrranose (produit AUBYGUM X2 vendu par la société SANOFI) ; les alginates de sodium (polymannuronate et guluronate) ; la cellulose et ses dérivés comme la méthylcellulose, le propylcellulose, l'hydroxypropylméthylcellulose ; les gommes de xanthane telles que celles constituées de D-glucose, D-mannose et l'acide D-glucuronique ; des polysaccharides riches en fucose comme le produit FUCOGEL 1000 vendu par la société SOLABIA.

Les compositions selon l'invention peuvent également contenir des synergistes de mousses tels que des alcanolamides comme les monoéthanolamides et dialcanolamides et plus particulièrement les monoisopropanolamides, les diéthanolamides ; les alkylamidodiméthylamines ; des dérivés d'oxydes d'amine comme les oxydes d'alkyldiméthylamine, les oxydes d'alkyldihydroxyéthylamine, les oxydes d'alkylamidométhyl-amine, les oxydes d'alkylamidohydroxyéthylamine, les oxydes d'alkylamido-hydroxyéthylamine. Ils sont présents, de préférence dans des concentrations inférieures ou égales à 10 % en poids par rapport au poids total de la composition.

Les compositions conformes à l'invention peuvent également contenir dans la phase huileuse un ou plusieurs actifs cosmétiques et/ou dermatologiques lipophiles tels que par exemples des agents anti-radicaux libres, des céramides ; des additifs de formulation lipophiles tels que des conservateurs, des antioxydants, des huiles parfumées.

Les compositions selon l'invention peuvent contenir en plus dans le milieu aqueux des actifs cosmétiques et/ou dermatologiques hydrophiles couramment utilisés tels que les polyols comme la glycérine, le tri-chloro-2,4,4'-hydroxy-2'-diphényléther, le sel de monoéthanolamine de l'hydroxy-1 méthyl-4 tri-méthylpentyl-6 pyridone-2 (ou piroctone olamine) tel que le produit OCTOPYROX vendu par la société HOECHST, des alpha-hydroxyacides, des agents kératolytiques comme l'urée, l'acide salicylique et ses dérivés tels que l'acide n-octanoyl 5-salicylique, des anti-bactériens, antiséborrhéiques, antipelliculaires. Elles peuvent contenir des additifs de formulation classiques tels que des pigments, des nacres, des charges minérales ou organiques telles le talc, le kaolin, les poudres de silice ou de polyéthylène, les particules creuses thermoplastiques expansées telles que le produit EXPANCEL 550 DE 20 vendu par la société NOBEL CASCO.

Les compositions selon l'invention présentent de préférence, une viscosité allant de 1,5 à 6 Pa.s et plus préférentiellement allant de 2,5 à 4 Pa.s.

Les compositions selon l'invention sous forme de crème produisent au contact de l'eau des mousses suffisamment abondantes.

Un autre objet de l'invention consiste en une composition cosmétique ou dermatologique de nettoyage de la peau, des muqueuses, des cheveux ou du cuir chevelu, constituée d'une crème moussante telle que définie ci-dessus.

Les crèmes moussantes de l'invention peuvent être utilisées comme base de produits de nettoyage/démaquillage de la peau, des yeux et/ou du visage.

Les crèmes moussantes de l'invention peuvent également être utilisées comme bases de produits de nettoyage et de soin des cheveux ou du cuir chevelu tels que des shampooings deux-en-un.

Les crèmes moussantes de l'invention peuvent également être utilisées comme bases de produits d'hygiène et de soin de la peau et/ou du corps tels que des crèmes de douche deux-en-un.

Un autre objet de l'invention consiste en un procédé de nettoyage de la peau, des muqueuses, le cuir chevelu caractérisé par le fait qu'on applique la crème moussante sur la peau, les muqueuses, le cuir chevelu ou les cheveux en présence d'eau et qu'on élimine la mousse formée et les résidus de salissures par un simple rinçage à l'eau.

Bien entendu, l'homme de l'art veillera à choisir le ou les éventuels composés complémentaires et/ou leurs quantités de manière telle que les propriétés avantageuses attachées intrinsèquement à l'association ternaire conforme à l'invention ne soient pas, ou substantiellement pas, altérées par la ou les adjonctions envisagées.

Les exemples qui suivent sont destinés à illustrer l'invention sans pour autant en limiter la portée.

## EXEMPLES

Dans tous les exemples de crèmes moussantes qui suivent, on a mesuré l'abondance des mousses produites selon la méthode suivante :

On place dans un bécher haut de forme de 400 ml, 2,5 g de composition à tester diluée dans 50 ml d'eau.

On forme une mousse par agitation de la composition en solution pendant 6 minutes à 1320 tours/minute au moyen d'un appareil TURBOTEST 10-14 RAYNERI muni d'une tige centripète de diamètres 30-40-55 cm ; celle-ci étant placée de manière excentrée à gauche dans le bécher.

On transvase ensuite le produit obtenu dans une éprouvette de 500 ml avec 2,5 ml d'erreur.

On mesure le volume du produit obtenu (mousse) à t = 0 , t = 3 minutes et t = 5 minutes.

Si l'on obtient avec la composition testée par cette méthode de mesure de volume de mousse, soit un volume inférieur à 150 ml soit une décomposition de la mousse après quelques minutes de pose, les propriétés moussantes de la composition testée sont considérées comme médiocres et inexploitables.

**Exemples 1 à 7**

| EXEMPLES | 1 | 2 | 3 | 4 | 5 | 6 | 7 |
|---|---|---|---|---|---|---|---|
| GELIFIANTS : | | | | | | | |
| CARBOPOL 980(R) | 0,9 g | 0,9 g | 1,1 g | - | 0,9 g | - | - |
| SYNTHALEN K(R) | - | - | - | - | - | 1 g | - |
| PAS 5193(R) | - | - | - | - | - | - | 6 g |
| SALCARE SC 95(R) | - | - | - | 4 g | - | - | |
| Gélifiant(s) Additifs | | | | | | | |
| AUBIGUM X2(R) | - | - | 0,1 g | - | - | - | - |
| TENSIOACTIFS NON IONIQUES : | | | | | | | |
| CHIMEXANE NF(R) | 5 g | 5 g | 5 g | 5 g | 5 g | 5 g | 5 g |
| Tensioactif de formule(I) | | | | | | | |
| S.L.M. S.P.G 120(R) | - | - | - | - | - | - | 2 g |
| NIKKOL HCO-60(R) | - | - | - | - | 1 g | - | - |
| CHARGES : | | | | | | | |
| EXPANCEL 551 DE 20(R) | - | - | 0,75 g | - | - | - | - |
| HUILE (S) : | | | | | | | |
| Huile de vaseline | - | - | - | - | 50 g | - | - |
| Fraction liq. de beurre de carité | 25 g | - | - | 25 g | - | - | 5 g |
| Huile de sésame | - | - | 20 g | - | - | - | - |
| Huile de colza | - | 25 g | - | - | - | 10 g | - |
| Triglycérides des acides caprylique/caprique | - | - | 3 g | - | - | - | - |
| ACTIFS | | | | | | | |
| Piroctone olamine | 0,2 g | - | 0,2 g | - | - | - | - |
| Acide lactique | - | - | - | - | - | - | 0,2 g |
| Acide n-octanoyl 5-salicylique | - | - | 0,2 g | - | - | - | - |
| Soude | 0,18 g | 0,18 g | 0,18 g | - | 0,18 g | 0,16 g | - |
| Glycérine | 6 g | 6 g | 6 g | 6 g | 8 g | - | 3 g |
| Eau q.s.p. | 100 g | 100 g | 100 g | 100 g | 100 g | 100 g | 100 g |
| Conservateur(s) q.s. | | | | | | | |
| Antioxydant(s) q.s. | | | | | | | |
| Volumes de mousse (ml) | 255 | 240 | 200 | 260 | 210 | 255 | 265 |

Les compositions des exemples 1 à 7 ont des propriétés moussantes satisfaisantes.

**Exemples 8 à 14**

| | 8 | 9 | 10 | 11 | 12 | 13 | 14 |
|---|---|---|---|---|---|---|---|
| GELIFIANT | | | | | | | |
| CARBOPOL 980$^{(R)}$ | 0,9 g | 0,9 g | 1,1 g | - | 0,9 g | - | - |
| SYNTHALEN K$^{(R)}$ | - | - | - | - | - | 1 g | - |
| PAS 5193$^{(R)}$ | - | - | - | - | - | - | 6 g |
| SALCARE SC 95$^{(R)}$ | - | - | - | 4 | - | - | - |
| Gélifiant(s) Add. | | | - | | | | |
| AUBIGUM X2$^{(R)}$ | - | - | 0,1 g | - | - | - | - |
| TENSIOACTIFS NON IONIQUES | | | | | | | |
| PLANTAREN 2000$^{(R)}$(*) | 10 g | 10 g | 10 g | 10 g | 10 g | 10 g | 10 g |
| Tensioactif de formule (II) | | | | | | | |
| S.L.M. S.P.G 120$^{(R)}$ | - | - | - | - | - | - | 2 g |
| NIKKOL HCO-60$^{(R)}$ | - | - | - | - | 1 g | - | - |
| CHARGES : | | | | | | | |
| EXPANCEL 551 DE 20$^{(R)}$ | | | | 0,75 g | - | - | - |
| HUILE (S) : | | | | | | | |
| Fraction liq. de beurre de carité | 25 g | - | - | 25 g | 50 g | - | 5 g |
| Huile de sésame | - | - | 20 g | - | - | - | - |
| Huile de colza | - | 25 g | - | | - | 10 g | - |
| Triglycérides des acides caprylique/caprique | - | - | 3 g | - | - | | - |
| ACTIFS | | | | | | | |
| Piroctone olamine | 0,2 g | - | 0,2 g | - | - | - | 0,2 g |
| Acide lactique | - | - | - | - | - | - | - |
| Acide n-octanoyl 5-salicylique | - | - | 0,2 g | - | - | - | - |
| Soude | 0,18 g | 0,18 g | 0,18 g | - | 0,18 g | 0,16 g | - |
| Glycérine | 6 g | 6 g | 6 g | 6 g | 8 g | - | 3 g |
| Eau q.s.p. | 100 g | 100 g | 100 g | 100 g | 100 g | 100 g | 100 g |
| Conservateur(s) q.s. | | | | | | | |
| Antioxydant(s) q.s. | | | | | | | |
| Volumes de mousse (ml) | 255 | 240 | 200 | 260 | 210 | 255 | 265 |

(*) matière première à 50% de matière active

Les compositions des exemples 8 à 14 ont des propriétés moussantes satisfaisantes.

**EXEMPLES COMPARATIFS**

On étudie l'abondance de la mousse obtenue à partir de différentes crèmes contenant différentes huiles présentant différentes valeurs de paramètres de solubilité dA et dD selon HANSEN. On compare les compositions de l'invention contenant comme phase huileuse une huile satisfaisant aux relations $15,5 \leq dD \leq 18$ et $0 \leq dA \leq 6,5$ à des compositions contenant comme phase huileuse une huile ne satisfaisant pas à ces conditions. Les volumes de mousses sont mesurés selon la méthode définie ci-dessus.

Les compositions testées ont pour formulation :

| | |
|---|---|
| - tensio-actif non-ionique de formule (I) CHIMEXANE NF | 5,0 g |
| - huile de paramètres dA, dD | 25,0 g |
| - agent gélifiant homopolymère acrylique réticulé CARBOPOL 980 | 0,9 g |
| - eau q.s.p. | 100,0 g |

Les huiles utilisées sont :

- l'isohexadecane
- le palmitate d'éthyl-2-hexyle
- le stéarate d'octyl-2-dodécyle
- l'érucate d'octyle-2-dodécyle
- l'isostéarate d'isostéaryle
- le benzoate d'octyl-2 dodecyle
- l'huile de jojoba
- l'huile de sésame
- les triglycérides d'acides caprique/caprylique
- l'octyldodécanol.

Les résultats sont résumés dans le tableau suivant :

| Huile utilisée dans la composition testée | dD $(J/cm^3)^{1/2}$ | dA $(J/cm^3)^{1/2}$ | Volume de mousse (ml) |
|---|---|---|---|
| Isohexadecane | 15,32 | 0 | 105 |
| Erucate d'octyl-2 dodécyle | 16,36 | 3,21 | 265 |
| Huile de jojoba | 16,33 | 3,33 | 265 |
| Stéarate d'octyl-2 dodécyle | 16,40 | 3,37 | 260 |
| Isostéarate d'isostéaryle | 16,31 | 3,46 | 245 |
| Benzoate d'octyl-2 dodécyle | 17,08 | 4,21 | 220 |
| Palmitate d'éthyl-2 hexyle | 16,20 | 4,23 | 190 |
| Huile de sésame | 16,36 | 4,85 | 260 |
| Triglycérides d'acides caprique/caprylique | 16,64 | 6,69 | 70 |
| Octyl-dodécanol | 16,36 | 7,69 | 65 |

Les résultats obtenus montrent que les phases constituées d'huiles ne répondant pas aux conditions déterminées sur les paramètres de solubilité selon HANSEN à savoir $15,5 \leq dD \leq 18$ et $0 \leq dA \leq 6,5$, ne permettent pas d'obtenir des crèmes présentant des propriétés moussantes satisfaisantes c'est le cas de l'isohexadécane, des triglycérides d'acides caprique/caprylique et de l'octyldodécanol.

**Revendications**

1. Emulsion huile-dans-eau moussante sous forme de crème, caractérisée par le fait qu'elle contient dans un milieu aqueux au moins :

a) 5 à 20%, en poids par rapport au poids total de la composition, d'un système tensio-actif exclusivement non-ionique contenant au moins un tensio-actif non-ionique moussant ;

b) 10 à 50%, en poids par rapport au poids total de la composition, d'une phase huileuse comportant au moins une huile non-soluble dans l'eau dont les paramètres de solubilité dD, dP, et dH selon l'espace de solubilité de HANSEN satisfont aux conditions suivantes :

$$15,5 \ (J/cm^3)^{1\!/\!2} \leq dD \leq 18,0 \ (J/cm^3)^{1\!/\!2}$$

$$et \ 0 \ (J/cm^3)^{1\!/\!2} \leq dA \leq 6,5 \ (J/cm^3)^{1\!/\!2}$$

$$avec \ dA = (dP^2 + dH^2)^{1\!/\!2}$$

c) comme agent gélifiant, au moins un homopolymère ou copolymère réticulé formé à partir d'au moins un monomère, cationique ou anionique, à insaturation éthylénique et d'un agent de réticulation à polyinsaturation éthylénique, à l'exception des copolymères comportant une fraction majoritaire d'au moins un acide oléfiniquement insaturé en $C_3$-$C_6$ et une fraction minoritaire d'au moins un ester d'un acide oléfiniquement insaturé en $C_3$-$C_6$ avec un alcool en $C_{10}$-$C_{30}$.

2. Emulsion selon la revendication 1, caractérisée par le fait qu'elle comprend :

a) 5 à 10%, en poids par rapport au poids total de la composition, d'un système tensio-actif exclusivement non-ionique contenant au moins un tensio-actif non-ionique moussant ;

b) 10 à 30%, en poids par rapport au poids total de la composition, d'une phase huileuse comportant au moins une huile non-soluble dans l'eau dont les paramètres de solubilité sont tels que définis à la revendication 1.

3. Emulsion selon la revendication 1 ou 2, caractérisée par le fait que les tensio-actifs non-ioniques moussants ont un pouvoir moussant déterminé par une hauteur de mousse supérieure à 100 mm selon la méthode ROSS-MILES pour une solution de 0,1 % en poids dans l'eau distillée à 25° C.

4. Emulsion selon l'une quelconque des revendications précédentes, caractérisée par le fait que les tensio-actifs non-ioniques moussants sont choisis parmi :

(i) les composés de formule :

$$R—CHOH—CH_2—O\text{[}CH_2CHOH—CH_2O\text{]}_nH \qquad (I)$$

dans laquelle R désigne un radical ou un mélange de radicaux aliphatiques, cycloaliphatiques ou arylaliphatiques ayant de 7 à 21 atomes de carbone, les chaînes aliphatiques pouvant être saturées ou non-saturées, linéaires ou ramifiées et pouvant comporter des atomes de souffre ou d'oxygène et en particulier des groupements éther, thioether ou hydroxyméthylène ; n est un nombre quelconque, entier ou décimal supérieur à 1 et inférieur ou égal à 10 et désigne le degré de polymérisation ;

(ii) les composés de formule :

$$R^1 \ O(G)_p \qquad (II)$$

dans laquelle $R^1$ est une chaîne hydrocarbonée monovalente contenant de 1 à 30 atomes de carbone, G est un dérivé de saccharide contenant de 5 à 6 atomes de carbone et p est une valeur statistique moyenne allant de 1 à 6 ;

(iii) leurs mélanges.

5. Emulsion selon la revendication 4, caractérisée par le fait que les composés de formule (I) sont choisis parmi ceux pour lesquels R désigne un radical ou un mélange de radicaux alkyle en $C_9$-$C_{12}$ et n représente une valeur statistique allant de 3 à 4.

6. Emulsion selon la revendication 4, caractérisée par le fait que les composés de formule (II), sont ceux pour lesquels $R^1$ est un radical alkyle en $C_8$-$C_{16}$ et n vaut 1,4 ou 1,6.

7. Emulsion selon la revendication 4, caractérisée par le fait qu'elle contient en plus des tensio-actifs non-ioniques moussants choisis parmi les polydiméthylsiloxanes à groupements glucosides ou les dérivés de polydiméthylsiloxane à groupements alkylphosphobétaïnes.

8. Emulsion selon l'une quelconque des revendications 1 à 7, caractérisée par le fait qu'elle contient au moins une huile choisie parmi les huiles minérales, les huiles d'origine animale et les huiles de synthèse répondant aux conditions relatives aux paramètres dA et dD telles que définies dans la revendication 1.

9. Emulsion selon la revendication 8, caractérisée par le fait que l'huile est choisie parmi l'huile de paraffine, l'huile de vaseline, le perhydrosqualène, l'huile de jojoba, l'huile de sésame, l'huile de colza, le beurre de karité et sa fraction liquide, l'huile de Purcellin, le palmitate d'éthyl-2 hexyle, le stéarate d'octyl-2 dodécyle, l'érucate d'octyl-2 dodécyle, l'isostéarate d'isostéaryle et le benzoate d'octyl-2 dodecyle et l'isoparaffine (6-8 moles d'isobutylène) hydrogénée.

10. Emulsion selon l'une quelconque des revendications 1 à 9, caractérisée par le fait qu'elle contient au moins une huile dont les paramètres de solubilité dA et dD satisfont aux conditions suivantes :

$$16{,}0 \ (\text{J/cm}^3)^{\frac{1}{2}} \leq dD \leq 18{,}0 \ (\text{J/cm}^3)^{\frac{1}{2}}$$

$$\text{et } 0 \ (\text{J/cm}^3)^{\frac{1}{2}} \leq dA \leq 5{,}5 \ (\text{J/cm}^3)^{\frac{1}{2}}$$

11. Emulsion selon l'une quelconque des revendications 1 à 10, caractérisée par le fait que la phase huileuse comprend de 85 à 100 % en poids d'huile satisfaisant aux conditions relatives à dA et dD définies dans la revendication 1 ou 10 et de 0 à 15% en poids d'huile ne répondant pas à ces conditions ; les pourcentages étant déterminées par rapport au poids total de la phase huileuse.

12. Emulsion selon l'une quelconque des revendications 1 à 11, caractérisée par le fait que l'agent gélifiant est constitué d'un homopolymère ou copolymère réticulé comprenant au moins un monomère anionique choisi parmi l'acide (meth)acrylique, le (meth)acrylate d'ammonium, l'acide 2-acrylamido-2-méthylpropane-sulfonique ainsi que leurs sels.

13. Emulsion selon l'une quelconque des revendications 1 à 12, caractérisée par le fait que l'agent gélifiant est constitué d'un homopolymère ou copolymère réticulé comprenant au moins un monomère cationique choisi parmi les dialkylaminoalkyl(meth)acrylates, les dialkylaminoalkyl(meth)acrylamides ainsi que leurs sels quaternaires et leurs acides ; les radicaux alkyle étant, de préférence, en $C_1$-$C_4$.

14. Emulsion selon l'une quelconque des revendications 1 à 13, caractérisée par le fait que l'agent de réticulation à polyinsaturation éthylénique est choisi parmi le divinylbenzène, le tétrallyloxyéthane, l'éther diallylique, les éthers polyallyliques polyglycéryliques, les éthers allyliques polyglycéryliques, les éthers allyliques d'alcools de la série du sucre ; les composés choisis parmi le méthylène-bis acrylamide, l'éthylène-glycol du diméthacrylate, le di(meth)acrylamide, le cyanométhylacrylate, le vinyloxyéthyl(meth)acrylate et leurs sels métalliques.

15. Emulsion selon l'une quelconque des revendications 12 à 14 caractérisée par le fait que les copolymères réticulés comprennent un comonomère non-ionique choisi parmi l'acrylamide, le methacrylamide ou les esters d'acide (meth)acrylique en $C_{10}$-$c_{30}$.

16. Emulsion selon l'une quelconque des revendications 1 à 15, caractérisée par le fait que l'agent gélifiant est choisi parmi :

(a) les homopolymères d'acide acrylique réticulés par un éther allylique d'alcool de la série du sucre ;

(b) les copolymères réticulés d'acide 2-acrylamido 2-méthylpropane sulfonique partiellement ou totalement neutralisé et d'acrylamide ;

(c) les homopolymères réticulés d'acrylate d'ammonium ou les copolymères réticulés d'acrylate d'ammonium et d'acrylamide ;

(d) les homopolymères de diméthylamino éthyl-méthacrylate quaternisé par le chlorure de méthyle ou les copolymères de ce même monomère avec l'acrylamide.

**17.** Emulsion selon l'une quelconque des revendications 1 à 16, caractérisée par le fait que l'agent gélifiant est présent dans des concentrations en matière active allant de 0,8 à 8 % en poids par rapport au poids total de ladite émulsion.

**18.** Emulsion selon l'une quelconque des revendications 1 à 17, caractérisée par le fait qu'elle contient en plus des tensio-actifs non-ioniques émulsionnants.

**19.** Emulsion selon l'une quelconque des revendications 1 à 18, caractérisée par le fait qu'elle contient en plus au moins un agent épaississant ayant un seuil d'écoulement faible et un faible pouvoir de gélification.

**20.** Emulsion selon la revendication 19, caractérisée par le fait que l'agent épaississant est choisi parmi les galactomannanes ; les carraghénanes ; les alginates de sodium ; la cellulose et ses dérivés; les gommes de xanthane et les polysaccharides riches en fucose.

**21.** Emulsion selon l'une quelconque des revendications 1 à 20, caractérisée par le fait qu'elle contient en plus au moins un agent synergiste de mousse.

**22.** Emulsion selon la revendication 21, caractérisée par le fait que l'agent synergiste de mousse est choisi parmi les alcanolamides, les alkylamido-diméthylamines ; les dérivés d'oxydes d'amines.

**23.** Emulsion selon l'une quelconque des revendications 1 à 22, caractérisée par le fait qu'elle contient en plus des actifs cosmétiques ou dermatologiques lipophiles ou hydrophiles ; des antioxydants, des conservateurs, des parfums, des antibactériens, des antiseborrhéiques, des antipelliculaires, des pigments, des agents kératolytiques, des colorants, des nacres, des charges minérales ou organiques.

**24.** Emulsion selon l'une quelconque des revendications 1 à 23, caractérisée par le fait qu'elle présente une viscosité allant de 1,5 à 6 Pa.s.

**25.** Composition cosmétique ou dermatologique de nettoyage de la peau, des muqueuses, des cheveux ou du cuir chevelu, caractérisée par le fait qu'elle est constituée de l'émulsion huile-dans-eau selon l'une quelconque des revendications 1 à 23.

**26.** Utilisation de l'émulsion huile-dans-eau selon l'une quelconque des revendications 1 à 24, comme base pour produits de nettoyage/démaquillage de la peau, des yeux et/ou du visage.

**27.** Utilisation de l'émulsion huile-dans-eau selon l'une quelconque des revendications 1 à 24, comme base de produits de nettoyage et de soin des cheveux.

**28.** Utilisation de l'émulsion huile-dans-eau selon l'une quelconque des revendications 1 à 24, comme base de produits d'hygiène et de soin de la peau et/ou du corps.

**29.** Procédé cosmétique de nettoyage de la peau, des muqueuses, des cheveux ou du cuir chevelu, caractérisé par le fait qu'on applique la composition selon la revendication 25 sur la peau, les muqueuses, les cheveux ou le cuir chevelu, en présence d'eau et qu'on élimine la mousse formée et les résidus de salissures par un simple rinçage à l'eau.

**Claims**

**1.** Foaming oil-in-water emulsion in the form of a cream, characterized in that it contains, in an aqueous medium, at least:

a) 5 to 20% by weight, relative to the total weight of the composition, of an exclusively nonionic surfactant system comprising at least one foaming nonionic surfactant;
b) 10 to 50% by weight, relative to the total weight of the composition, of an oily phase containing at least one water-insoluble oil whose solubility parameters dD, dP and dH according to Hansen's solubility space satisfy the following conditions:

$$15.5(J/cm^3)^{1/2} \leq dD \leq 18.0 \ (J/cm^3)^{1/2}$$

$$\text{and } 0 \ (\text{J/cm}^3)^{\frac{1}{2}} \leq dA \leq 6.5 \ (\text{J/cm}^3)^{\frac{1}{2}}$$

$$\text{with } dA = (dP^2 + dH^2)^{\frac{1}{2}}$$

c) and, as gelling agent, at least one crosslinked homopolymer or copolymer formed from at least one cationic or anionic monomer containing ethylenic unsaturation and from a crosslinking agent containing polyethylenic unsaturation, with the exception of copolymers containing a majority fraction of at least one olefinically unsaturated $C_3$-$C_6$ acid and a minority fraction of at least one ester of an olefinically unsaturated $C_3$-$C_6$ acid with a $C_{10}$-$C_{30}$ alcohol.

2. Emulsion according to Claim 1, characterized in that it comprises:

a) 5 to 100% by weight, relative to the total weight of the composition, of an exclusively nonionic surfactant system comprising at least one foaming nonionic surfactant;
b) 10 to 30% by weight, relative to the total weight of the composition, of an oily phase containing at least one water-insoluble oil whose solubility parameters are as defined in Claim 1.

3. Emulsion according to Claim 1 or 2, characterized in that the foaming nonionic surfactants have a foaming power determined by a foam height of more than 100 mm according to the Ross-Miles method for a 0.1% by weight solution in distilled water at 25°C.

4. Emulsion according to any one of the preceding claims, characterized in that the foaming nonionic surfactants are chosen from:

(i) the compounds of formula:

$$R\text{—CHOH—CH}_2\text{—O}\{\text{CH}_2\text{CHOH—CH}_2\text{O}\}_n\text{H} \tag{I}$$

in which R denotes an aliphatic, cycloaliphatic or arylaliphatic radical or mixture of radicals having from 7 to 21 carbon atoms, it being possible for the aliphatic chains to be saturated or unsaturated, linear or branched and possibly to contain sulphur or oxygen atoms and in particular ether, thioether or hydroxymethylene groups; n is any integer or fraction greater than 1 and less than or equal to 10 and denotes the degree of polymerization;
(ii) the compounds of formula

$$R^1O(G)_p \tag{II}$$

in which $R^1$ is a monovalent hydrocarbon chain containing from 1 to 30 carbon atoms, G is a saccharide derivative containing from 5 to 6 carbon atoms and p is a statistical average value ranging from 1 to 6;
(iii) mixtures thereof.

5. Emulsion according to Claim 4, characterized in that the compounds of formula (I) are chosen from those for which R denotes a $C_9$-$C_{12}$ alkyl radical or mixture of radicals and n represents a statistical value ranging from 3 to 4.

6. Emulsion according to Claim 4, characterized in that the compounds of formula (II) are those for which $R^1$ is a $C_8$-$C_{16}$ alkyl radical and n is equal to 1.4 or 1.6.

7. Emulsion according to Claim 4, characterized in that it also contains foaming nonionic surfactants chosen from polydimethylsiloxanes containing glucoside groups or polydimethylsiloxane derivatives containing alkylphosphobetaine groups.

8. Emulsion according to any one of Claims 1 to 7, characterized in that it contains at least one oil chosen from mineral oils, oils of animal origin and synthetic oils corresponding to the conditions relating to the parameters dA and dD as defined in Claim 1.

9. Emulsion according to Claim 8, characterized in that the oil is chosen from liquid paraffin, liquid petrolatum, perhydrosqualene, jojoba oil, sesame oil, rapeseed oil, karite butter and the liquid fraction thereof, purcellin oil, 2-ethylhexyl palmitate, 2-octyldodecyl stearate, 2-octyldodecyl erucate, isostearyl isostearate and 2-octyldodecyl benzoate and hydrogenated isoparaffin (6_8 mol of isobutylene).

10. Emulsion according to any one of Claims 1 to 9, characterized in that it contains at least one oil whose solubility parameters dA and dD satisfy the following conditions:

$$16.0 \ (J/cm^3)^{1/2} \leq dD \leq 18.0 \ (J/cm^3)^{1/2}$$

$$\text{and } 0 \ (J/cm^3)^{1/2} \leq dA \leq 5.5 \ (J/cm^3)^{1/2}$$

11. Emulsion according to any one of Claims 1 to 10, characterized in that the oily phase comprises from 85 to 100 % by weight of oil which satisfies the conditions relating to dA and dD defined in Claim 1 or 10 and from 0 to 15% by weight of oil which does not correspond to these conditions; the percentages being determined relative to the total weight of the oily phase.

12. Emulsion according to any one of Claims 1 to 11, characterized in that the gelling agent consists of a crosslinked homopolymer or copolymer comprising at least one anionic monomer chosen from (meth)acrylic acid, ammonium (meth)acrylate, 2-acrylamido-2-methylpropanesulphonic acid and salts thereof.

13. Emulsion according to any one of Claims 1 to 12, characterized in that the gelling agent consists of a crosslinked homopolymer or copolymer comprising at least one cationic monomer chosen from dialkylaminoalkyl (meth)acrylates, dialkylaminoalkyl (meth)acrylamides and the quaternary salts and acids thereof; the alkyl radicals preferably being $C_1$-$C_4$.

14. Emulsion according to any one of Claims 1 to 13, characterized in that the crosslinking agent containing polyethylenic unsaturation is chosen from divinylbenzene, tetraallyloxyethane, diallyl ether, polyallyl polyglyceryl ethers, allyl polyglyceryl ethers, allyl ethers of alcohols from the sugar series; the compounds chosen are from methylenebis(acrylamide), ethylene glycol dimethacrylate, di(meth)acrylamide, cyanomethyl acrylate, vinyloxyethyl (meth)acrylate and metal salts thereof.

15. Emulsion according to any one of Claims 12 to 14, characterized in that the crosslinked copolymers comprise a nonionic comonomer chosen from acrylamide, methacrylamide or $C_{10}$-$C_{30}$ esters of (meth)acrylic acid.

16. Emulsion according to any one of Claims 1 to 15, characterized in that the gelling agent is chosen from:

   (a) acrylic acid homopolymers crosslinked with an allyl ether of an alcohol from the sugar series;
   (b) crosslinked copolymers of 2-acrylamido-2-methylpropanesulphonic acid which is partially or totally neutralized and of acrylamide;
   (c) crosslinked homopolymers of ammonium acrylate or crosslinked copolymers of ammonium acrylate and of acrylamide;
   (d) homopolymers of dimethylaminoethyl methacrylate quaternized with methyl chloride or the copolymers of this same monomer with acrylamide.

17. Emulsion according to any one of Claims 1 to 16, characterized in that the gelling agent is present in active material concentrations ranging from 0.8 to 8% by weight relative to the total weight of the said emulsion.

18. Emulsion according to any one of Claims 1 to 17, characterized in that it also contains emulsifying nonionic surfactants.

19. Emulsion according to any one of Claims 1 to 18, characterized in that it also contains at least one thickening agent having a low flow threshold and a low gelling power.

20. Emulsion according to Claim 19, characterized in that the thickening agent is chosen from galactomannans; carrageenans; sodium alginates; cellulose and derivatives thereof; xanthan gums and fucose-rich polysaccharides.

21. Emulsion according to any one of Claims 1 to 20, characterized in that it also contains at least one foam synergist.

22. Emulsion according to Claim 21, characterized in that the foam synergist is chosen from alkanolamides, alkylamidodimethylamines and amine oxide derivatives.

23. Emulsion according to any one of Claims 1 to 22, characterized in that it also contains lipophilic or hydrophilic cosmetic or dermatological active agents; antioxidants, preserving agents, fragrances, anti-bacterial agents, antiseborrhoeic agents, antidandruff agents, pigments, keratolytic agents, dyes, pearlescent agents and inorganic or organic fillers.

24. Emulsion according to any one of Claims 1 to 23, characterized in that it has a viscosity ranging from 1.5 to 6 Pa s.

25. Cosmetic or dermatological composition for cleansing the skin, the mucous membranes, the hair or the scalp, characterized in that it consists of the oil-in-water emulsion according to any one of Claims 1 to 23.

26. Use of the oil-in-water emulsion according to any one of Claims 1 to 24, as a base for products for the cleansing of/removal of make-up from the skin, the eyes and/or the face.

27. Use of the oil-in-water emulsion according to any one of Claims 1 to 24, as a base for cleansing and care products for the hair.

28. Use of the oil-in-water emulsion according to any one of Claims 1 to 24, as a base for hygiene and care products for the skin and/or the body.

29. Cosmetic process for cleansing the skin, the mucous membranes, the hair or the scalp, characterized in that the composition according to Claim 25 is applied to the skin, the mucous membranes, the hair or the scalp, in the presence of water, and that the foam formed and the dirt residues are removed by simple rinsing with water.

**Patentansprüche**

1. Schäumende Öl-in-Wasser-Emulsion in Form einer Creme, dadurch gekennzeichnet, daß sie in einem wäßrigen Medium mindestens folgende Bestandteile enthält:

   a) 5 bis 20 Gew.-%, bezogen auf das Gesamtgewicht der Zusammensetzung, eines ausschließlich nicht-ionogenen oberflächenaktiven Systems, das mindestens ein schäumendes nicht-ionogenes Tensid enthält;
   b) 10 bis 50 Gew.-%, bezogen auf das Gesamtgewicht der Zusammensetzung, einer öligen Phase, die mindestens ein in Wasser nicht-lösliches Öl enthält, deren Löslichkeitsparameter dD, dP und dH gemäß dem Löslichkeitsraum nach HANSEN folgende Bedingungen erfüllen:

   $$15,5 \ (J/cm^3)^{1\!/\!2} \leq dD \leq 18,0 \ (J/cm^3)^{1\!/\!2}$$

   $$\text{und } 0 \ (J/cm^3)^{1\!/\!2} \leq dA \leq 6,5 \ (J/cm^3)^{1\!/\!2}$$

   $$\text{mit } dA = (dP^2 + dH^2)^{1\!/\!2};$$

   c) als gelbildendes Mittel mindestens ein vernetztes Homopolymeres oder Copolymeres, das aus mindestens einem kationischen oder anionischen Monomeren mit einer ungesättigten ethylenischen Bindung und einem mehrfach ethylenisch ungesättigten Vernetzungsmittel gebildet worden ist, mit der Ausnahme von Copolymeren, die einen überwiegenden Anteil mindestens einer olefinisch ungesättigten $C_3$-$C_6$-Säure und einen untergeordneten Anteil mindestens eines Esters einer olefinisch ungesättigten $C_3$-$C_6$-Säure mit einem $C_{10}$-$C_{30}$-Alkohol enthalten.

2. Emulsion nach Anspruch 1, dadurch gekennzeichnet, daß sie folgende Bestandteile umfaßt:

   a) 5 bis 10 Gew.-%, bezogen auf das Gesamtgewicht der Zusammensetzung, eines ausschließlich nicht-ionogenen oberflächenaktiven Systems, das mindestens ein schäumendes nicht-ionogenes Tensid enthält;
   b) 10 bis 30 Gew.-%, bezogen auf das Gesamtgewicht der Zusammensetzung, einer öligen Phase, die mindestens ein in Wasser nicht-lösliches Öl enthält, deren Löslichkeitsparameter der Definition in Anspruch 1 entsprechen.

3. Emulsion nach Anspruch 1 oder 2, dadurch gekennzeichnet, daß die schäumenden, nicht-ionischen Tenside ein nach der Methode von ROSS-MILES für eine Lösung von 0,1 Gew.-% in destilliertem Wasser von 25°C aufgrund der Schaumhöhe bestimmtes Schaumbildungsvermögen von mehr als 100 mm aufweisen.

4. Emulsion nach einem der vorstehenden Ansprüche, dadurch gekennzeichnet, daß die schäumenden, nicht-ionogenen Tenside ausgewählt sind unter:

(i) Verbindungen der Formel:

$$R\text{---}CHOH\text{---}CH_2\text{---}O\text{--}[\text{--}CH_2CHOH\text{---}CH_2O\text{--}]_n\text{--}H \qquad (I)$$

worin R einen aliphatischen, cycloaliphatischen oder arylaliphatischen Rest mit 7 bis 21 Kohlenstoffatomen oder ein Gemisch derartiger Reste bedeutet, wobei die aliphatischen Ketten gesättigt oder ungesättigt sowie linear oder verzweigt sein können und Schwefel- oder Sauerstoffatome und insbesondere Ether-, Thioether- oder Hydroxymethylengruppen enthalten können; n eine beliebige ganze Zahl oder Dezimalzahl, die größer als 1 und kleiner oder gleich 10 ist, bedeutet und den Polymerisationsgrad bezeichnet;
(ii) Verbindungen der Formel:

$$R^1O(G)_p \qquad (II)$$

worin $R^1$ eine einwertige Kohlenwasserstoffkette mit 1 bis 30 Kohlenstoffatomen bedeutet, G ein Saccharidderivat mit 5 bis 6 Kohlenstoffatomen bedeutet und p einen statistischen Mittelwert von 1 bis 6 bedeutet;
(iii) Gemische davon.

5. Emulsion nach Anspruch 4, dadurch gekennzeichnet, daß die Verbindungen der Formel (I) unter Verbindungen ausgewählt sind, in denen R einen $C_9$-$C_{12}$-Alkylrest oder ein Gemisch von derartigen Alkylresten bedeutet und n einen statistischen Wert von 3 bis 4 bedeutet.

6. Emulsion nach Anspruch 4, dadurch gekennzeichnet, daß die Verbindungen der Formel (II) unter Verbindungen ausgewählt sind, in denen $R^1$ einen $C_8$-$C_{16}$-Alkylrest bedeutet und n einen Wert von 1,4 bis 1,6 hat.

7. Emulsion nach Anspruch 4, dadurch gekennzeichnet, daß sie zusätzlich schäumende, nicht-ionogene Tenside enthält, die unter Polydimethylsiloxanen mit Glucosidgruppen oder Polydimethylsiloxanderivaten mit Alkylphosphobetaingruppen ausgewählt sind.

8. Emulsion nach einem der Ansprüche 1 bis 7, dadurch gekennzeichnet, daß sie mindestens ein Öl enthält, daß unter Mineralölen, Ölen tierischen Ursprungs und synthetischen Ölen ausgewählt ist, die den Bedingungen bezüglich der Parameter dA und dD gemäß der Definition in Anspruch 1 entsprechen.

9. Emulsion nach Anspruch 8, dadurch gekennzeichnet, daß das Öl ausgewählt ist unter: Paraffinöl, Vaselinöl, Perhydrosqualen, Jojobaöl, Sesamöl, Rapsöl, Schibutter und seine flüssige Fraktion, Purcellinöl, 2-Ethylhexylpalmitat, 2-Octyldodecylstearat, 2-Octyldodecylerucat, Isostearylisostearat und 2-Octyldodecylbenzoat sowie hydriertes Isoparaffin (6-8 Mol Isobutylen).

10. Emulsion nach einem der Ansprüche 1 bis 9, dadurch gekennzeichnet, daß sie mindestens ein Öl enthält, dessen Löslichkeitsparameter dA und dD die folgenden Bedingungen erfüllen:

$$16,0 \ (J/cm^3)^{\frac{1}{2}} \leq dD \leq 18,0 \ (J/cm^3)^{\frac{1}{2}}$$

$$\text{und } 0 \ (J/cm^3)^{\frac{1}{2}} \leq dA \leq 5,5 \ (J/cm^3)^{\frac{1}{2}}.$$

11. Emulsion nach einem der Ansprüche 1 bis 10, dadurch gekennzeichnet, daß die Ölphase 85 bis 100 Gew.-% eines Öls enthält, das die Bedingungen bezüglich dA und dD gemäß der Definition in den Ansprüchen 1 oder 10 erfüllt und 0 bis 15 Gew.-% eines Öls, das diese Bedingungen nicht erfüllt, enthält, wobei die prozentualen Anteile auf das Gesamtgewicht der öligen Phase bezogen sind.

12. Emulsion nach einem der Ansprüche 1 bis 11, dadurch gekennzeichnet, daß das gelbildende Mittel aus einem vernetzten Homopolymeren oder Copolymeren besteht, das mindestens ein anionisches Monomeres enthält, das unter (Meth)acrylsäure, Ammonium(meth)acrylat, 2-Acrylamido-2-methylpropansulfonsäure und deren Salzen ausgewählt ist.

13. Emulsion nach einem der Ansprüche 1 bis 12, dadurch gekennzeichnet, daß das gelbildende Mittel aus einem vernetzten Homopolymeren oder Copolymeren besteht, das mindestens ein kationisches Monomeres umfaßt, das unter Dialkylaminoalkyl(meth)acrylaten, Dialkylaminoalkyl(meth)acrylamiden sowie deren quaternären Salzen und deren Säuren ausgewählt ist, wobei es sich bei den Alkylresten vorzugsweise um $C_1$-$C_4$-Reste handelt.

14. Emulsion nach einem der Ansprüche 1 bis 13, dadurch gekennzeichnet, daß das ethylenisch mehrfach ungesättigte Vernetzungsmittel ausgewählt ist unter: Divinylbenzol, Tetraallyloxyethan, Diallylether, Polyallylpolyglycerylether, Allylpolyglycerlyether, Allylether von Alkoholen der Zuckerreihe; Verbindungen aus der Gruppe Methylenbisacrylamid, Ethylenglycoldimethacrylat, Di(meth)acrylamid, Cyanomethacrylat, Vinyloxyethyl(meth)acrylat und deren Metallsalze.

15. Emulsion nach einem der Ansprüche 12 bis 14, dadurch gekennzeichnet, daß die vernetzten Copolymeren ein nichtionogenes Comonomeres umfassen, das unter Acrylamid, Methacrylamid oder $C_{10}$-$C_{30}$-(Meth)acrylsäureestern ausgewählt ist.

16. Emulsion nach einem der Ansprüche 1 bis 15, dadurch gekennzeichnet, daß das gelbildende Mittel ausgewählt ist unter:

(a) Homopolymeren von Acrylsäure, die mit einem Allylether eines Alkohols der Zuckerreihe vernetzt sind;
(b) vernetzte Copolymere von partiell oder vollständig neutralisierter 2-Acrylamido-2-methylpropansulfonsäure und Acrylamid;
(c) vernetzte Homopolymere von Ammoniumacrylat oder vernetzte Comonomere von Ammoniumacrylat und Acrylamid;
(d) Homopolymere von Dimethylaminoethylmethacrylat, das mit Methylchlorid quaternisiert ist, oder Copolymere des gleichen Monomeren mit Acrylamid.

17. Emulsion nach einem der Ansprüche 1 bis 16, dadurch gekennzeichnet, daß das gelbildende Mittel in Wirkstoffkonzentrationen von 0,8 bis 8 Gew.-%, bezogen auf das Gesamtgewicht der Emulsion, vorhanden ist.

18. Emulsion nach einem der Ansprüche 1 bis 17, dadurch gekennzeichnet, daß sie zusätzlich emulgierende, nichtionogene Tenside enthält.

19. Emulsion nach einem der Ansprüche 1 bis 18, dadurch gekennzeichnet, daß sie zusätzlich mindestens ein Verdickungsmittel enthält, das einen geringen Fließschwellenwert und ein schwaches Gelbildungsvermögen aufweist.

20. Emulsion nach Anspruch 19, dadurch gekennzeichnet, daß das Verdickungsmittel ausgewählt ist unter: Galactomannanen; Carraghenanen; Natriumalginaten; Cellulose und ihren Derivaten; Xanthangummen; und Polysacchariden mit einem hohen Fucosegehalt.

21. Emulsion nach einem der Ansprüche 1 bis 20, dadurch gekennzeichnet, daß sie zusätzlich ein synergistisches Schaumbildungsmittel enthält.

22. Emulsion nach Anspruch 21, dadurch gekennzeichnet, daß das synergistische Schaumbildungsmittel ausgewählt ist unter: Alkanolamiden, Alkylamidodimethylaminen und Derivate von Aminoxiden.

23. Emulsion nach einem der Ansprüche 1 bis 22, dadurch gekennzeichnet, daß sie zusätzlich folgendes enthält: lipophile oder hydrophile kosmetische oder dermatologische Wirkstoffe, Antioxidantien, Konservierungsmittel, Parfums, antibakterielle Mittel, Mittel gegen Seborrhoe, Antischuppenmittel, Pigmente, keratolytische Mittel, farbgebende Mittel, Glanzmittel, anorganische oder organische Füllstoffe.

24. Emulsion nach einem der Ansprüche 1 bis 23, dadurch gekennzeichnet, daß sie eine Viskosität von 1,5 bis 6 Pa.s aufweist.

25. Kosmetische oder dermatologische Zusammensetzung zur Reinigung der Haut, der Schleimhäute, der Haare oder der Kopfhaut, dadurch gekennzeichnet, daß sie aus einer Öl-in-Wasser-Emulsion nach einem der Ansprüche 1 bis 23 besteht.

26. Verwendung der Öl-in-Wasser-Emulsion nach einem der Ansprüche 1 bis 24 als Grundlage für Reinigungs/Abschmink-Produkte der Haut, der Augen und/oder des Gesichts.

**27.** Verwendung der Öl-in-Wasser-Emulsion nach einem der Ansprüche 1 bis 24 als Grundlage für Reinigungs- und Pflegeprodukte für Haare.

**28.** Verwendung der Öl-in-Wasser-Emulsion nach einem der Ansprüche 1 bis 24 als Grundlage für Hygiene- und Pflegeprodukte der Haut und/oder des Körpers.

**29.** Kosmetisches Verfahren zur Reinigung der Haut, der Schleimhäute, der Haare oder der Kopfhaut, dadurch gekennzeichnet, daß man die Zusammensetzung nach Anspruch 25 auf die Haut, die Schleimhäute, die Haare oder die Kopfhaut in Gegenwart von Wasser aufbringt und den gebildeten Schaum und die Schmutzreste durch einfaches Spülen mit Wasser entfernt.